# EUROPEAN PATENT APPLICATION

(11) **EP 4 249 497 A1**
(43) Date of publication of application: **27.09.2023**
(21) Application number: 22163579.0
(22) Date of filing: 22.03.2022
(51) Int. Cl.: C07K 7/06, A61K 38/08, A61P 29/00, A61K 35/618

(54) **NOVEL SST4 SELECTIVE AGONISTS AS NON-OPIOID ANALGESICS**

(71) Applicant: Københavns Universitet, 1165 Copenhagen K (DK); University of Utah Research Foundation, Salt Lake City, UT 84112 (US)
(72) Inventor: Bjørn-Yoshimoto, Walden, 1165 Copenhagen K (DK); Ramiro, Iris Bea L., 1165 Copenhagen K (DK); Koch, Thomas Lund, 1165 Copenhagen K (DK); Safavi-Hemami, Helena, 1165 Copenhagen K (DK)
(74) Representative: Plougmann Vingtoft a/s

(57) **Abstract**

The present invention relates to novel somatostatin 4 (SST4) selective agonists as non-opioid analgesics. In particular, the present invention relates to conopeptides having strong SST4-selectivity for use in treatment of pain or inflammation.

## Description

### Field of the invention

The present invention relates to novel somatostatin receptor 4 (SST4) selective agonists as non-opioid analgesics. In particular, the present invention relates to conopeptides having strong SST4-selectivity for use in treatment of pain or inflammation.

The project leading to this application has received funding from the European Research Council (ERC) under the European Union's Horizon 2020 research and innovation program (grant agreement No 949830).

### Background of the invention

The inventors of the present invention have discovered that a largely unexplored, deep-water lineage of fish-hunting cone snails of the *Asprella* clade, in particular the two species *Conus rolani* and *Conus neocostatus,* uses a relatively slow predation strategy, i.e. the *Asprella* envenomation takes 1-3 hours from the first strike to engulfment of prey. This unexpected observation led the inventors to investigate venom components that potentially facilitate the hitherto unknown behavior observed. By combining the behavioral observations of prey-capture by the cone snail predator with behavioral changes elicited by the action of individual venom components in mice, a novel class of analogs of the vertebrate hormone somatostatin was discovered.

Further, somatostatin (SS) is a peptide hormone with diverse physiological roles. By investigating a deep-water clade of fish-hunting cone snails, the inventors showed that predator-prey evolution has generated a diverse set of SS analogs, each optimized to elicit specific systemic physiological effects in prey. The increased metabolic stability, distinct SS receptor (SST) activation profiles, and chemical diversity of the venom analogs make them suitable leads for therapeutic application, including pain and inflammation.

WO 2017/139845 A1 discloses inter alia conopeptides in the context of treating pain, including chronic pain, inflammatory pain, neuropathic pain, visceral pain, breakthrough pain, bipolar disorder, Alzheimer's disease, Parkinson's disease, producing analgesia or enhancement of opiate analgesia. The conopeptides of WO 2017/139845 A1 are not disclosed in the context of SST4 agonist properties.

Olivera B. M. (Annual report dated October 2020, prepared for the U.S. Army Medical Research and Development Command Fort Detrick, Maryland 21702-5012 "Novel Strategies for Accelerating Non-Opioid Drug Discovery" University of Utah) discloses e.g. venoms collected from *Conus rolani*, including peptides, such as Cont-Ro4, shown to induce analgesia in the formalin pain model.

While opioids are very efficient in the management of acute nociceptive pain, they fall short with chronic pain. Additionally, opioids have the disastrous pharmacologic profile of dependence after prolonged use and carry a high risk of respiratory depression.

Hence, the provision of improved non-opioid based analgesic having their effect outside of the central nervous system (CNS), i.e. outside the brain and spinal cord would be highly advantageous.

### Summary of the invention

The invention relates to peptides from cone snails, hereinafter referred to as conopeptides, that activate a certain type of somatostatin receptor (mainly SST4), and are some of the most, if not the most selective compounds ever described for this receptor.

The conopeptides of the invention provide analgesia that is (1) via a non-opioid based mechanism and (2) by actions outside of the CNS. The conopeptides of the invention are intended for pain management, where they can alleviate pain, while avoiding the addictive properties of the current gold standard, opioids. The conopeptides of the invention are also intended for use in the treatment of inflammation.

Activation of SST4, and to some degree somatostatin receptor 1 (SST1) with the conopeptides of the present invention, has been shown to have analgesic effects, as well as anti-inflammatory effects.

The inventors recently described a novel class of cyclical somatostatin receptor activating conopeptides. One of those identified, consomatin Ro1 (also referred to as "Cont-Rol"), showed a low potency SST1 and SST4 (~5µM) activation and analgesic effects as strong as morphine, but longer lasting, when injected peripherally in mice. Notably, this only occurred when injected peripherally, not when injected intracranially, suggesting a non-CNS-based target, and suggesting that the peptide does not have to cross the blood-brain-barrier to induce analgesia.

Based on the Cont-Rol findings, the inventors looked for additional "somatostatin-like" conopeptides, and found e.g. one of the peptides this patent application concerns, here called HSH-109. Initial tests showed it to be (A) more potent and (B) more selective for SST4 than Cont-Rol (concentration-response curve shown under "5. Developmental stage").

The apparent potencies observed for the initial peptide (HSH-109) are in the low nanomolar range for SST4, and in the low micromolar range for SST1. Interestingly, the conopeptide did not significantly activate the SST2, SST3 or SST5 at these concentrations.

Thus, one aspect of the invention relates to a conopeptide comprising or consisting of a sequence of the following consecutive amino acids: cysteine, D-tryptophan, lysine, phenylalanine, glycine and cysteine.

Another aspect of the present invention relates to a conopeptide comprising or consisting of the amino acid sequence:
Xaa1, Xaa2, Xaa3, Xaa4, Xaa5, Xaa6 Xaa7, Xaa8, Xaa9 and Xaa10, wherein
Xaa1 is selected from proline, leucine, tyrosine, lysine, methionine or is absent;
Xaa2 is selected from valine, phenylalanine, lysine, tryptophan, aspartate, proline or is absent;
Xaa3 is cysteine;
Xaa4 is tryptophan, tyrosine, phenylalanine, or histidine;
Xaa5 is lysine or arginine;
Xaa6 is phenylalanine;
Xaa7 is glycine, alanine, threonine, serine, valine, asparagine, leucine, isoleucine, or is absent;
Xaa8 is cysteine;
Xaa9 is proline, tryptophan, methionine, leucine, isoleucine or is absent;
Xaa10 is leucine, proline, or is absent.

Yet another aspect of the present invention is to provide a conopeptide according to any of, for use as a medicament in a human or mammal subject and/or for use in treatment of pain or inflammation in a human or mammal subject.

Still another aspect of the present invention relates to the use of an isolated conopeptide selected from the group consisting of: (a) a peptide selected from the group consisting of peptides comprising amino acid sequences set forth in SEQ ID NOs: 1-14; and (b) analogs and derivatives of the peptide in (a) as a non-opioid analgesic or an anti-inflammatory agent.

### Brief description of the figures

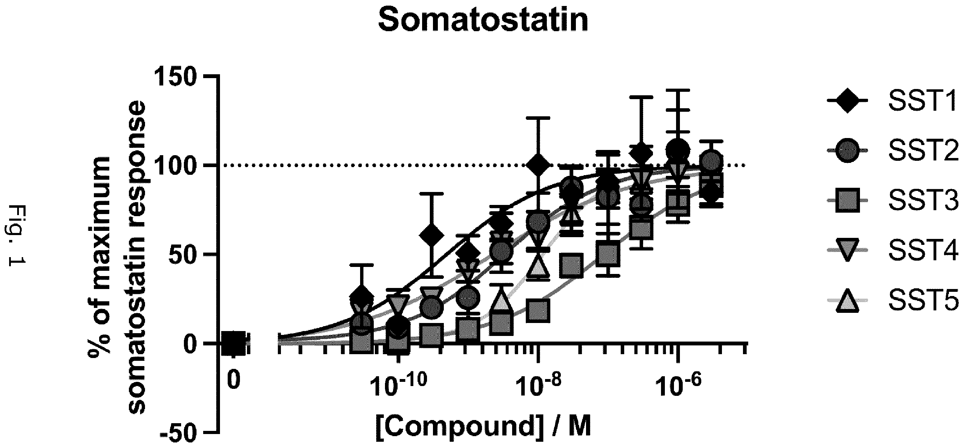

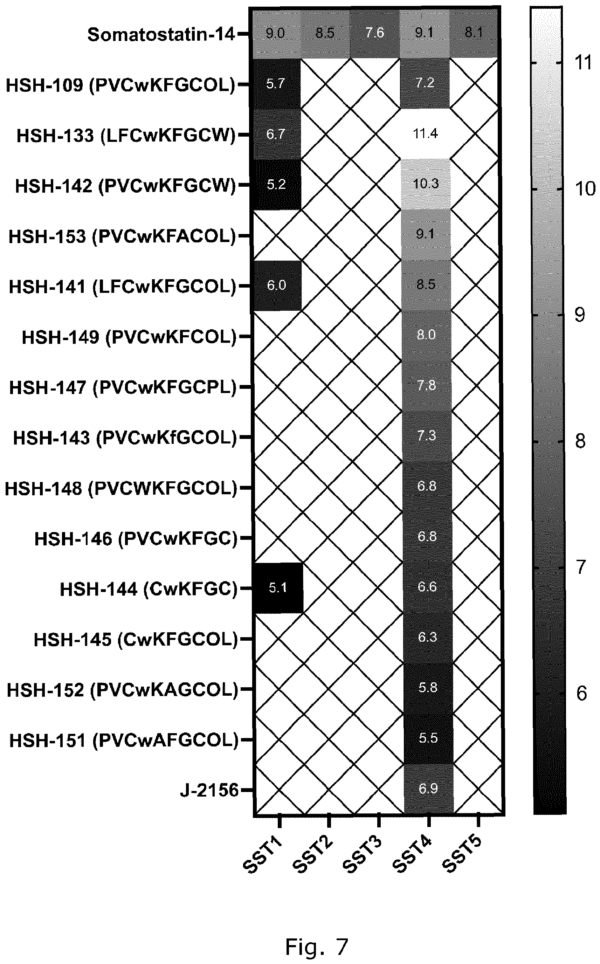

Figure 1 shows representative concentration-response curves of human somatostatin-14 (SS-14) at the five human somatostatin receptors (SST1-5) in the PRESTO-Tango β-arrestin recruitment assay (a standardized GPCR activation assay). Each curve is normalized to maximum somatostatin response at the given receptor. EC₅₀ values are shown in figure 7.

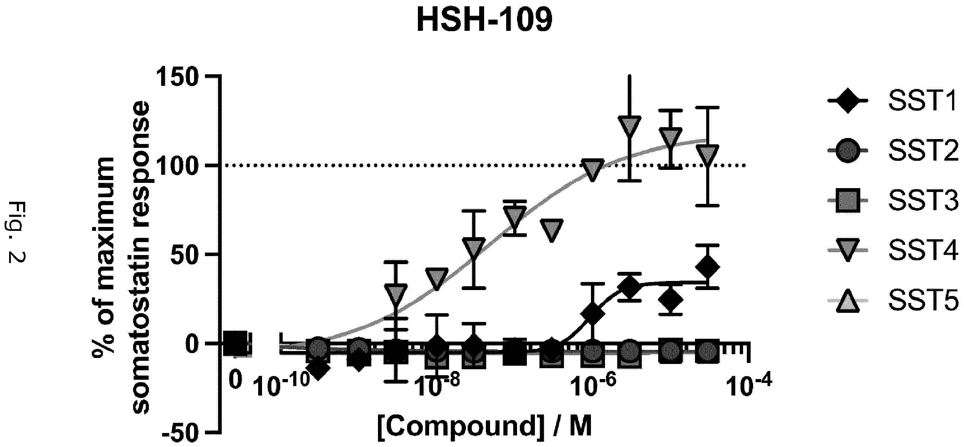

Figure 2 shows representative concentration-response curves of HSH-109 (SEQ ID NO 1) at the five human somatostatin receptors (SST1-5) in the PRESTO-Tango β-arrestin recruitment assay (a standardized GPCR activation assay). Each curve is normalized to maximum somatostatin response. EC₅₀ values are shown in figure 7.

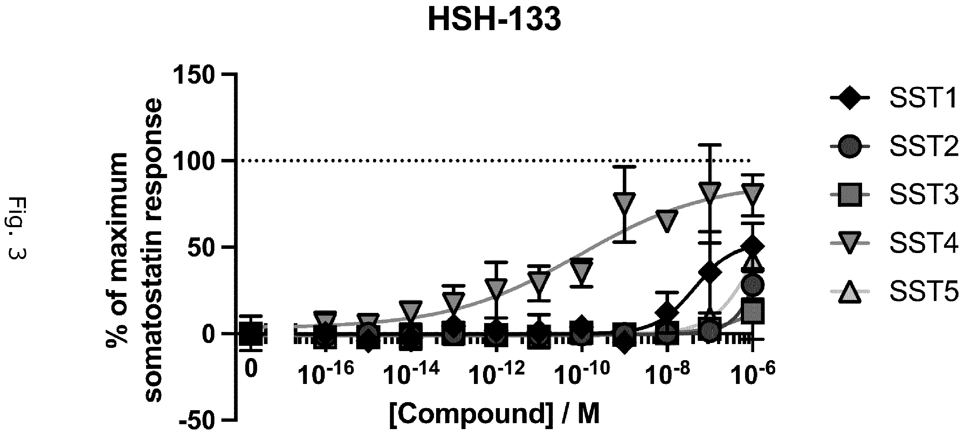

Figure 3 shows representative concentration-response curves of HSH-133 (SEQ ID NO 2) at the five human somatostatin receptors (SST1-5) in the PRESTO-Tango β-arrestin recruitment assay (a standardized GPCR activation assay). Each curve is normalized to maximum somatostatin response. EC₅₀ values are shown in figure 7.

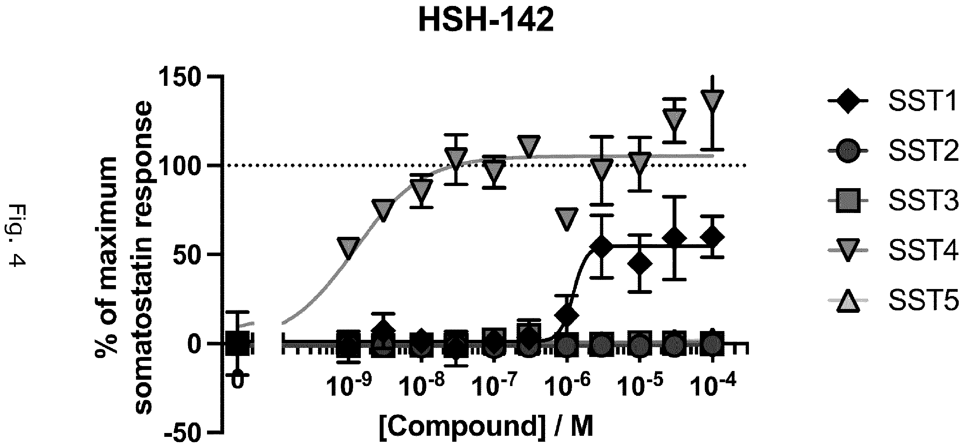

Figure 4 shows representative concentration-response curves of HSH-142 (SEQ ID NO 3) at the five human somatostatin receptors (SST1-5) in the PRESTO-Tango β-arrestin recruitment assay (a standardized GPCR activation assay). Each curve is normalized to maximum somatostatin response. EC₅₀ values are shown in figure 7.

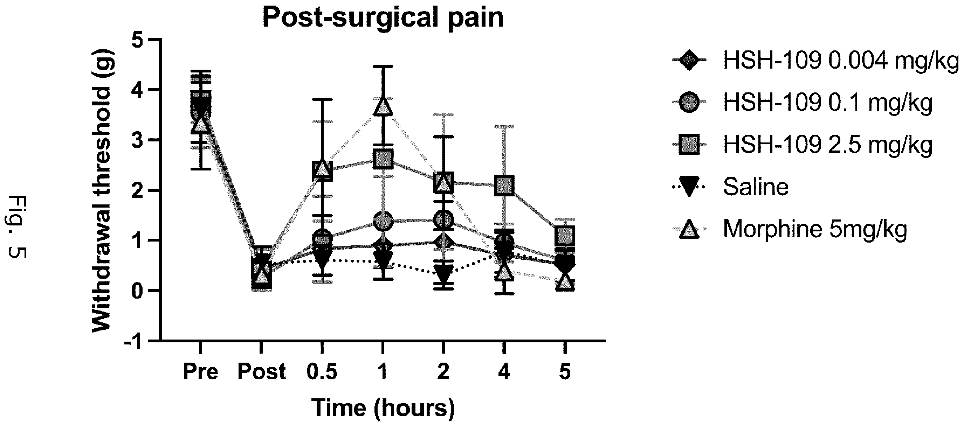

Figure 5 shows the effect of a single injection of 0.004 - 2.5 mg/kg of HSH-109 in a post-surgical pain model in mice (Von Frey hairs) using normal saline as a negative control, and 5 mg/kg morphine as a positive control. Figure 5 is response over time post-surgery. Six mice were used in each condition, and the datapoints represent mean values, with the error bars being standard deviation.

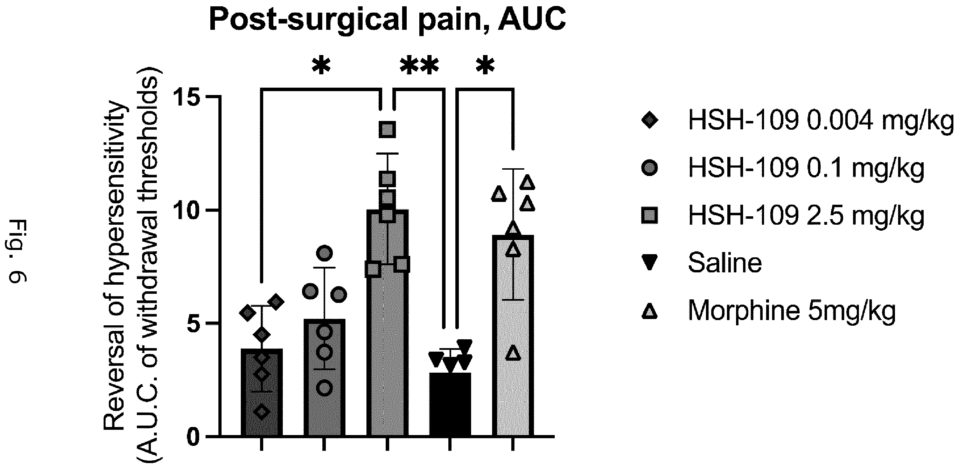

Figure 6 shows the effect of a single injection of 0.004 - 2.5 mg/kg of HSH-109 in a post-surgical pain model in mice (Von Frey hair measurement) using normal saline as a negative control, and 5 mg/kg morphine as a positive control. Figure 6 is a summary of the area under the curve (AUC) of data shown in figure 5, showing each mouse as a single datapoint in the overlayed scatterplot. Six mice were used in each condition, the bars show the mean and the error bars represent 95 % confidence intervals. Kruskal-Wallis test with Dunn's post-test was performed for statistical comparisons. ^{∗} indicates p<0.05, ^{∗∗} indicates P<0.01.
Figure 7 shows a heatmap of the mean EC50 values of each compound tested at the five somatostatin receptors in the PRESTO-Tango β-arrestin recruitment assay (a standardized GPCR activation assay). The top is somatostatin-14, bottom is the SST4 selective small molecule, J-2156. All peptides are labelled with their name and amino acid sequence in parenthesis. Lower case letters denote D-amino acids, "O" indicates hydroxyproline. An "X" indicates that the curve was not completed at the highest concentration used, and thus a reliable EC50 value could not be extracted, indicating that the compound was less potency at a given receptor, than at receptors with a listed EC₅₀ value.

The present invention will now be described in more detail in the following.

### Detailed description of the invention

Prior to discussing the present invention in further details, the following terms and conventions will first be defined:

### Embodiments

In one embodiment of the present invention there is provided a conopeptide comprising or consisting of a conserved core sequence of the following consecutive amino acids: cysteine, D-tryptophan, lysine, phenylalanine, glycine and cysteine.

In another embodiment of the present invention there is provided a conopeptide comprising or consisting of a conserved core sequence of the following consecutive amino acids: cysteine, D-tryptophan, lysine, phenylalanine, glycine and cysteine, for use as a medicament.

In still another embodiment of the present invention there is provided a conopeptide comprising or consisting of a conserved core sequence of the following consecutive amino acids: cysteine, D-tryptophan, lysine, phenylalanine, glycine and cysteine, for use in treatment of pain or inflammation in a human or mammal subject.

In still another embodiment of the present invention there is provided a conopeptide comprising or consisting of an amino acid sequence of SEQ ID NO 1-14.

In still another embodiment of the present invention there is provided a conopeptide comprising or consisting of an amino acid sequence of SEQ ID NO 1-14, for use as a medicament.

In still another embodiment of the present invention there is provided a conopeptide comprising or consisting of an amino acid sequence of SEQ ID NO 1-14, for use in treatment of pain or inflammation in a human or mammal subject.

In still another embodiment of the present invention there is provided a conopeptide comprising or consisting of the amino acid sequence:
Xaa1, Xaa2, Xaa3, Xaa4, Xaa5, Xaa6 Xaa7, Xaa8, Xaa9 and Xaa10, wherein
Xaa1 is selected from proline, leucine, tyrosine, lysine, methionine, hydroxyproline, or is absent;
Xaa2 is selected from valine, phenylalanine, lysine, tryptophan, aspartate, proline, hydroxyproline, or is absent;
Xaa3 is cysteine or (i) cysteine making disulfide bonds to the cysteine of Xaa8 or (ii) is replaced by an amino acid that can form dicarba bonds, other amino acids, or penicillamine that can form bonds to Xaa8;
Xaa4 is tryptophan, tyrosine, phenylalanine, or histidine;
Xaa5 is lysine or arginine;
Xaa6 is phenylalanine or D-phenylalanine;
Xaa7 is glycine, alanine, threonine, serine, valine, asparagine, leucine, isoleucine, or is absent;
Xaa8 is cysteine or (i) cysteine making disulfide bonds to the cysteine of Xaa8 or (ii) is replaced by an amino acid that can form dicarba bonds, other amino acids, or penicillamine that can form bonds to Xaa3;
Xaa9 is proline, hydroxyproline, tryptophan, methionine, leucine, isoleucine, or is absent; Xaa10 is leucine, proline, hydroxyproline, or is absent.

In still another embodiment of the present invention there is provided a conopeptide comprising or consisting of the amino acid sequence:
Xaa1, Xaa2, Xaa3, Xaa4, Xaa5, Xaa6 Xaa7, Xaa8, Xaa9 and Xaa10, wherein
Xaa1 is selected from proline, leucine, tyrosine, lysine, methionine, hydroxyproline, or is absent;
Xaa2 is selected from valine, phenylalanine, lysine, tryptophan, aspartate, proline, hydroxyproline, or is absent;
Xaa3 is cysteine or (i) cysteine making disulfide bonds to the cysteine of Xaa8 or (ii) is replaced by an amino acid that can form dicarba bonds, other amino acids, or penicillamine that can form bonds to Xaa8;
Xaa4 is tryptophan, tyrosine, phenylalanine, or histidine;
Xaa5 is lysine or arginine;
Xaa6 is phenylalanine or D-phenylalanine;
Xaa7 is glycine, alanine, threonine, serine, valine, asparagine, leucine, isoleucine, or is absent;
Xaa8 is cysteine or (i) cysteine making disulfide bonds to the cysteine of Xaa8 or (ii) is replaced by an amino acid that can form dicarba bonds, other amino acids, or penicillamine that can form bonds to Xaa3;
Xaa9 is proline, hydroxyproline, tryptophan, methionine, leucine, isoleucine, or is absent;
Xaa10 is leucine, proline, hydroxyproline, or is absent;
for use as a medicament.

In still another embodiment of the present invention there is provided a conopeptide comprising or consisting of the amino acid sequence:
Xaa1, Xaa2, Xaa3, Xaa4, Xaa5, Xaa6 Xaa7, Xaa8, Xaa9 and Xaa10, wherein
Xaa1 is selected from proline, leucine, tyrosine, lysine, methionine, hydroxyproline, or is absent;
Xaa2 is selected from valine, phenylalanine, lysine, tryptophan, aspartate, proline, hydroxyproline, or is absent;
Xaa3 is cysteine or (i) cysteine making disulfide bonds to the cysteine of Xaa8 or (ii) is replaced by an amino acid that can form dicarba bonds, other amino acids, or penicillamine that can form bonds to Xaa8;
Xaa4 is tryptophan, tyrosine, phenylalanine, or histidine;
Xaa5 is lysine or arginine;
Xaa6 is phenylalanine or D-phenylalanine;
Xaa7 is glycine, alanine, threonine, serine, valine, asparagine, leucine, isoleucine, or is absent;
Xaa8 is cysteine or (i) cysteine making disulfide bonds to the cysteine of Xaa8 or (ii) is replaced by an amino acid that can form dicarba bonds, other amino acids, or penicillamine that can form bonds to Xaa3;
Xaa9 is proline, hydroxyproline, tryptophan, methionine, leucine, isoleucine, or is absent;
Xaa10 is leucine, proline, hydroxyproline, or is absent;
for use in treatment of pain or inflammation in a human or mammal subject.

In still another embodiment of the present invention there is provided a method of treating or prevention of pain, such as acute, post-surgical, inflammatory, chronic, visceral or breakthrough pain, comprising administering to a subject in need thereof an effective amount of a conopeptide according to the present invention.

In still another embodiment, the present invention provides a method for the treatment or prevention of inflammation comprising administering to a subject in need thereof an effective amount of a conopeptide according to the present invention.

In still another embodiment, the present invention provides a use of an isolated conopeptide selected from the group consisting of: (a) a peptide selected from the group consisting of peptides comprising amino acid sequences set forth in SEQ ID NOs: 1-14; or (b) analogs and derivatives of any of the peptides in (a) as a non-opioid analgesic or an anti-inflammatory agent.

While the peptide according to the invention may be the sole active ingredient administered to the subject, the administration of other active ingredients with said peptide is within the scope of the invention. For example, the peptide could be administered with one or more therapeutic agents.

As will be readily appreciated by those skilled in the art, the route of administration will depend on the nature of the condition and the human or mammal to be treated. As is generally known in the art, in the preparation of any formulation containing the peptide actives, care should be taken to ensure that the activity of the peptide is not destroyed in the process and that the peptide is able to reach its site of action without being destroyed. Also, as is well known in the art some formulations are given to enhance the activity, e.g. by improve the availability or increase the solubility of a compound. In some circumstances it may be necessary to protect the peptide by means known in the art, such as, for example, micro encapsulation. Similarly, the route of administration chosen should be such that the peptide reaches its site of action.

Another aspect of the present invention relates to prodrugs which may be metabolized to any of the conopeptides of the invention (SEQ ID NOs 1-14) once taken up into the body.

In some embodiments, a peptide or a composition comprising a peptide of the invention may be administered orally, dermally, topically, or subcutaneously.

In some embodiments, a peptide or a composition comprising a peptide of the invention may be administered in doses of 1-10 mg/kg, such as 2.5 mg/kg (mice) or 0.1-1 mg/kg (humans). Other dosages could be envisaged.

### Definitions

### Amino acid

The term "amino acid" is used herein in its broadest sense and may refer to compounds having an amino group and a carboxylic acid group. The amino acids incorporated into the peptides of the present invention may be D- or L-forms of proteogenic or naturally occurring amino acids, or may be D- or L-forms of non-proteogenic or non-naturally occurring amino acids. As referred to herein, the term extends to synthetic amino acids and analogues thereof, including salts, isomers, tautomers, esters, and N-methylated amino acids.

### Conopeptide

The term conopeptide as used herein refers to any peptide found in any snails of the genus Conus. Since both modifications and exact cleavage sites for the mature peptides are not readily predictable from the DNA or RNA sequence, "conopeptide" is used to cover any cleavage pattern or naturally occurring posttranslational modification that can arise, given an RNA or DNA sequence.

### Consomatin Ro1 (Cont-Rol)

Consomatin Ro1 is a venom peptide and as used herein refers to a mimetic of the vertebrate hormone somatostatin. Consomatin Ro1 represents the first SS-like peptide that was isolated from cone snail venom.

### Consomatin

The term consomatin as used herein refers to any conopeptide, as defined above, that belongs to the so-called C-toxin gene superfamily and mimics the sequence of somatostatin by having two cysteines that form a disulfide bond and a tryptophan-lysine (WK) motif between the two cysteines that form the disulfide.

### Exon capture

When used herein "exon capture" refers to a method of sequencing the coding regions of a genome.

### Pain

When used herein "pain" refers to and encompasses, but is not limited to, inflammatory pain, neuropathic pain, chronic pain, visceral pain, breakthrough pain, acute pain, nociceptive pain, and radicular pain.

### Peptide or protein

The terms "peptide" and "protein" are used herein interchangeably in their broadest sense to refer to oligomers of two or more amino acids, including isolated, synthetic or recombinant peptides. These terms apply to amino acid polymers in which one or more amino acid residues is/are a synthetic non-naturally occurring amino acid, such as a chemical analogue of a corresponding naturally occurring amino acid, as well as to naturally occurring amino acid polymers. These terms do not exclude modifications, for example, glycosylations, acetylations, phosphorylations and the like. Soluble forms of the subject peptides are particularly useful. Included within the definition are, for example, peptides containing one or more analogues of an amino acid including, for example, unnatural amino acids or peptides with substituted linkages.

### Somatostatin (SS)

SS is a highly conserved vertebrate peptide hormone that, in humans, is predominantly secreted in the brain, pancreas, and gastrointestinal tract where it functions as an inhibitor of secretion and cell proliferation. SS was first discovered as the main inhibitor of growth hormone (GH)-release from the anterior pituitary gland and has since been associated with many additional biological functions, including the inhibition of pancreatic hormone secretion, neuronal signaling, pain, and inflammation.

### SST4 (or SST1) "selective" and "selectivity"

The terms "selective" and "selectivity" as used herein refers to an activation on one subtype of receptor (e.g. SST4) with a potency at least 100-fold higher than for the subtype it displays "selectivity" over. If not compared explicitly to any one specific somatostatin receptor, it is assumed that it is compared to all other human somatostatin receptor subtypes.

### SST4 (or SST1) "preferring" and "preference" or similar

The terms "preferring" and "preference" as used herein refers to an activation on one subtype of receptor (e.g. SST4) with a potency between 10 and 100-fold higher than for the subtype it displays "preference" over. If not compared explicitly to any one specific somatostatin receptor, it is assumed that it is compared to all other human somatostatin receptor subtypes.

### Examples

### Example 1 - peptide synthesis and purification

The discovery of Cont-Rol led the inventors to search for other SS-like peptides in cone snail venom.

In order to specifically identify candidates that likely activate the SST, the inventors searched for sequences that belong to the C-toxin gene family and had at least five amino acid residues that are known to be important for SST activation by human somatostatin.

These amino acids included two cysteines, a phenylalanine, a tryptophan, and a lysine. The inventors searched cone snail transcriptome, genome and exon capture data and identified a DNA sequence from cone snail exon capture data that they performed initial in vitro tests on.

The inventors have also designed the first round of analogs based on substituting amino acid residues found in other peptides in the conopeptide inventory and performed initial tests of the first of these analogs.

The peptides according to the present invention may be prepared using standard peptide synthetic methods followed by oxidative disulfide bond formation, for example as discussed in the present Example.

### Peptide synthesis and purification

HSH-109 and HSH-109 analogs were synthesized using solid phase peptide synthesis on an automated peptide synthesizer. Peptides were cleaved from resins and oxidized in the presence of dimethylsulfoxide (DMSO). Folded peptides were purified using high-performance liquid chromatography (HPLC) and dried in a lyophilizer for storage.

### Example 2 - Concentration-response

Concentration-response curves of somatostatin-14 (SS-14), HSH-109, HSH-133, and HSH-142 at the five human somatostatin receptors (SST1-5) in the PRESTO-Tango β-arrestin recruitment assay (a standardized GPCR activation assay) are shown in figures 1-4.

Somatostatin-14 curves are shown in figure 1, while the conopeptide compounds are shown in figures 2-4. Each graph represents one compound, showing each SST1-5, normalized to maximum SS response.

### Example 3 - In vivo mouse model testing

*Withdrawal threshold test with HSH-109 in mouse model in vivo.*

### Measurement of tactile sensory thresholds - Von Frey test

The assessment of tactile sensory thresholds was determined by measuring the withdrawal response to probing the plantar surface of the hind paw with a series of calibrated filaments (von Frey) 24 hours post-surgery.

Each filament was applied perpendicularly to the plantar surface of the paw of a mouse held in suspended wire mesh cages. The "up and down" method was used to identify the mechanical force required for a paw withdrawal. Data were analyzed with the nonparametric method of Dixon, as described by Chaplan et al. (S. R. Chaplan, F. W. Bach, J. W. Pogrel, J. M. Chung, T. L. Yaksh, Quantitative assessment of tactile allodynia in the rat paw. Journal of neuroscience methods 53, 55-63 40 (1994*).).*

Results are expressed as the mean withdrawal threshold from six animals in each condition (figures 5-6).

The inventors observed a robust analgesic effect at 2.5 mg/kg in the mouse model tested. At present, it is not clear whether saturating doses was used or if higher dose would give increased effect. Expected doses for mice would be 1-10 mg/kg and for humans 0.1-1 mg/kg if injected.

### Example 4 - Inflammatory testing in vitro

Inflammation will be tested in the priority year in primary peritoneal macrophage cells isolated from mice. Cell cultures will be subject to lipopolysaccharide challenge in the presence or absence of test peptides (e.g. HSH-109) for 1 - 2 days, whereafter supernatant will be analyzed for inflammatory, as well as anti-inflammatory markers (e.g. TNFα, IL6, IL12, and MCP-1, as well as IL4 and IL10) using standard sandwich ELISA techniques.

### Example 5 - Inflammation and inflammation-induced pain testing in vivo

During the priority year, a rat model of osteoarthritis (e.g. the MMT, ACLT, or the monoiodoacetate model) will be used to assess (1) the inflammation in arthritic joints and (2) pain response associated with arthritis (e.g., mechanical allodynia). Rats will be injected intra peritoneally with test peptides (e.g. HSH-109).

The inflammation levels will be assessed by biochemical quantification of a range of typical inflammatory and anti-inflammatory biomarkers (e.g. TNFα, IFNγ, IL6, and IL10) by standard sandwich ELISA techniques. Likewise, biomarkers of collagen damage (e.g. CTX-II) will be assessed.

The mechanical allodynia will be performed using the Von Frey Hair technique, as described above, merely without performing any surgery.

### Sequence listing

*ST.25 standard definition*
(source WIPO, https://www.wipo.int/export/sites/www/standards/en/pdf/archives/03-25-01arc2009.pdf)

According to the ST.25 standard definition, "amino acids" are those L-amino acids commonly found in naturally occurring proteins and are listed in Table 1. Those amino acid sequences containing at least one D-amino acid are not intended to be embraced by this definition. Any amino acid sequence that contains post-translationally modified amino acids may be described as the amino acid sequence that is initially translated using the symbols shown in Table 2, with the modified positions, for example, hydroxylations or glycosylations. But these modifications shall not be shown explicitly in the amino acid sequence. Any peptide that can be expressed as a sequence using the symbols in Table 1, in conjunction with a description elsewhere to describe, for example, abnormal linkages, cross-links (for example, disulfide bridge) and end caps, non-peptidyl bonds, etc., is embraced by the ST.25 standard definition.

**Table 1 List of amino acids**

| **Amino acid** | **L- three letter code** | **D- three letter code** | **L-one letter code** | **D-one letter code** |
|---|---|---|---|---|
| Alanine | Ala | ala | A | a |
| Cysteine | Cys | cys | C | c |
| Aspartic Acid | Asp | asp | D | d |
| Glutamic Acid | Glu | glu | E | e |
| Phenylalanine | Phe | phe | F | f |
| Glycine | Gly | gly | G | g |
| Histidine | His | his | H | h |
| Isoleucine | Ile | ile | I | i |
| Lysine | Lys | lys | K | k |
| Leucine | Leu | leu | L | l |
| Methionine | Met | met | M | m |
| Asparagine | Asn | asn | N | n |
| Proline | Pro | pro | P | p |
| Glutamine | Gln | gln | Q | q |
| Arginine | Arg | arg | R | r |
| Serine | Ser | ser | S | s |
| Threonine | Thr | thr | T | t |
| Valine | Val | val | V | v |
| Tryptophan | Trp | trp | W | w |
| Tyrosine | Tyr | tyr | Y | y |
| Asp or Asn | Asx | asx | | |
| Glu or Gln | Glx | glx | | |
| unknown or other | Xaa | xaa | | |

| | | | | |
|---|---|---|---|---|
| f = D-phenylalanine (D-Phe, D-phenylalanine is the D-enantiomer of phenylalanine) w = D-tryptophan (D-Trp, D-tryptophan is the D-enantiomer of tryptophan) O = Hydroxyproline | | | | |

**Table 2**

| **Designated name(s)** | **SEQ ID NO.** | **ST.25-sequence format** | **Amino acid sequence of conopeptides of the invention (post-translationally modified)** |
|---|---|---|---|
| HSH-109 | SEQ ID NO. 1 | PVCWKFGCPL | PVCwKFGCOL |
| HSH-133 (HSH-109-[P1L,V2F,O9W, ΔL10]) | SEQ ID NO. 2 | LFCWKFGCW | LFCwKFGCW |
| HSH-142 (HSH-109-[O9W, ΔL10]) | SEQ ID NO. 3 | PVCWKFGCW | PVCwKFGCW |
| HSH-153 (HSH-109-[G7A]) | SEQ ID NO. 4 | PVCWKFACPL | PVCwKFACOL |
| HSH-141 (HSH-109-[P1L,V2F]) | SEQ ID NO. 5 | LFCWKFGCPL | LFCwKFGCOL |
| HSH-149 (HSH-109-[ΔG7]) | SEQ ID NO. 6 | PVCWKFCPL | PVCwKFCOL |
| HSH-147 (HSH-109-[O9P]) | SEQ ID NO. 7 | PVCWKFGCPL | PVCwKFGCPL |
| HSH-143 (HSH-109-[F6f]) | SEQ ID NO. 8 | PVCWKFGCPL | PVCwKfGCOL |
| HSH-148 (HSH-109-[w4W]) | SEQ ID NO. 9 | PVCWKFGCPL | PVCWKFGCOL |
| HSH-146 (HSH-109-[ΔO9,ΔL10]) | SEQ ID NO. 10 | PVCWKFGC | PVCwKFGC |
| HSH-144 (HSH-109-[ΔP1,ΔV2,ΔO9,ΔL10]) | SEQ ID NO. 11 | CWKFGC | CwKFGC |
| HSH-145 (HSH-109-[ΔP1,ΔV2]) | SEQ ID NO. 12 | CWKFGCPL | CwKFGCOL |
| HSH-152 (HSH-109-[F6A]) | SEQ ID NO. 13 | PVCWKAGCPL | PVCwKAGCOL |
| HSH-151 (HSH-109-[K5A]) | SEQ ID NO. 14 | PVCWAFGCPL | PVCwAFGCOL |

<110> University of Copenhagen
<120> Novel SST4 selective agonists as non-opioid analgesics
<130> 78230EP01
<160> 14
<170> BiSSAP 1.3.6

<210> 1
<211> 10
<212> PRT
<213> Cone snail
<223> HSH-109
<400> 1
Pro Val Cys Trp Lys Phe Gly Cys Pro Leu
1               5                   10

<210> 2
<211> 9
<212> PRT
<213> Cone snail
<223> HSH-133
<400> 2
Leu Phe Cys Trp Lys Phe Gly Cys Trp
1               5

<210> 3
<211> 9
<212> PRT
<213> Cone snail
<223> HSH-142
<400> 3
Pro Val Cys Trp Lys Phe Gly Cys Trp
1               5

<210> 4
<211> 10
<212> PRT
<213> Cone snail
<223> HSH-153
<400> 4

Pro Val Cys Trp Lys Phe Ala Cys Pro Leu
1               5                   10

<210> 5
<211> 10
<212> PRT
<213> Cone snail
<223> HSH-141
<400> 5
Leu Phe Cys Trp Lys Phe Gly Cys Pro Leu
1               5                   10

<210> 6
<211> 9
<212> PRT
<213> Cone snail
<223> HSH-149
<400> 6
Pro Val Cys Trp Lys Phe Cys Pro Leu
1               5

<210> 7
<211> 10
<212> PRT
<213> Cone snail
<223> HSH-147
<400> 7
Pro Val Cys Trp Lys Phe Gly Cys Pro Leu
1               5                   10

<210> 8
<211> 10
<212> PRT
<213> Cone snail
<223> HSH-143
<400> 8
Pro Val Cys Trp Lys Phe Gly Cys Pro Leu
1               5                   10

<210> 9
<211> 10
<212> PRT
<213> Cone snail
<223> HSH-148
<400> 9
Pro Val Cys Trp Lys Phe Gly Cys Pro Leu
1               5                   10

<210> 10
<211> 8
<212> PRT
<213> Cone snail
<223> HSH-146
<400> 10
Pro Val Cys Trp Lys Phe Gly Cys
1               5

<210> 11
<211> 6
<212> PRT
<213> Cone snail
<223> HSH-144
<400> 11
Cys Trp Lys Phe Gly Cys
1               5

<210> 12
<211> 8
<212> PRT
<213> Cone snail
<223> HSH-145
<400> 12
Cys Trp Lys Phe Gly Cys Pro Leu
1               5

<210> 13
<211> 10

<212> PRT
<213> Cone snail
<223> HSH-152
<400> 13
Pro Val Cys Trp Lys Ala Gly Cys Pro Leu
1               5                   10

<210> 14
<211> 10
<212> PRT
<213> Cone snail
<223> HSH-151
<400> 14
Pro Val Cys Trp Ala Phe Gly Cys Pro Leu
1               5                   10

### Abbreviations

ACLT: anterior cruciate ligament transection
AUC: area under the curve
CNS: central nervous system
Cont-Rol: consomatin Ro1 (a cyclical somatostatin receptor activating conopeptide)
CTX-II: Neo-epitope of MMP mediated degradation of type II collagen
EC₅₀: The concentration that elicits half of the maximal response.
IL: Interleukin
INFγ: Interferon gamma
GH: growth hormone
MCP-1: Monocyte chemoattractant protein-1
PRT: protein
MMT: Medial meniscus transection
SS: somatostatin
SST: somatostatin receptor
SST1: somatostatin receptor 1
SST2: somatostatin receptor 2
SST3: somatostatin receptor 3
SST4: somatostatin receptor 4
SST5: somatostatin receptor 5
TNFa: Tumor necrosis factor alpha

## Claims

1. A conopeptide comprising or consisting of a sequence of the following consecutive amino acids: cysteine, D-tryptophan, lysine, phenylalanine, glycine and cysteine.

2. A conopeptide comprising or consisting of the amino acid sequence:
Xaa1, Xaa2, Xaa3, Xaa4, Xaa5, aa6 Xaa7, Xaa8, Xaa9 and Xaa10, wherein
Xaa1 is selected from proline, leucine, tyrosine, lysine, methionine, or is absent;
Xaa2 is selected from valine, phenylalanine, lysine, tryptophan, aspartate, proline, or is absent;
Xaa3 is cysteine;
Xaa4 is tryptophan, tyrosine, phenylalanine, or histidine;
Xaa5 is lysine or arginine;
Xaa6 is phenylalanine;
Xaa7 is glycine, alanine, threonine, serine, valine, asparagine, leucine, isoleucine, or is absent;
Xaa8 is cysteine;
Xaa9 is proline, tryptophan, methionine, leucine, isoleucine, or is absent;
Xaa10 is leucine, proline, or is absent.

3. The conopeptide according to claim 2, wherein the cysteine in Xaa3 is (i) cysteine making disulfide bonds to the cysteine of Xaa8 or (ii) is replaced by other amino acids that can form bonds to Xaa8.

4. The conopeptide according to claim 3, wherein the replacement under (ii) is replacement with penicillamine.

5. The conopeptide according to any of claims 2-4, wherein the disulfide bond between the Xaa3 and Xaa8 is replaced by a dicarba bond.

6. The conopeptide according to any of claims 2-5, wherein the tryptophan in Xaa4 is D-tryptophan.

7. The conopeptide according to any of claims 2-6, wherein the phenylalanine in Xaa6 is D-phenylalanine.

8. The conopeptide according to any of claims 2-7, wherein the proline in Xaa9 is hydroxyproline.

9. An isolated conopeptide of SEQ ID NOs 1-14.

10. An isolated conopeptide according to claim 9, wherein any tryptophan is D-tryptophan and any proline is hydroxyproline.

11. The conopeptide according to any of claims 1-10, wherein said conopeptide is a cyclical conopeptide.

12. The conopeptide according to any of claims 1-11, for use as a medicament.

13. The conopeptide according to any of claims 1-11, for use in treatment of pain or inflammation in a human or mammal subject.

14. The conopeptide for use according to claim 12 or 13, wherein said conopeptide is administered into the human or mammal subject via an oral, dermal, topical or subcutaneously route.

15. Use of an isolated conopeptide selected from the group consisting of:
(a) a peptide selected from the group consisting of peptides comprising amino acid sequences set forth in SEQ ID NOs: 1-14; or
(b) analogs and derivatives of any of the peptides in (a)
as a non-opioid analgesic or an anti-inflammatory agent.
